Europäisches Patentamt

European Patent Office

Office européen des brevets

(11) Publication number: **0 561 568 A1**

## (12) EUROPEAN PATENT APPLICATION

(21) Application number: 93301893.9

(51) Int. Cl.⁵: **C07C 263/18,** C08G 18/70

(22) Date of filing: 12.03.93

(30) Priority: **16.03.92 JP 58320/92**

(43) Date of publication of application:
**22.09.93 Bulletin 93/38**

(84) Designated Contracting States:
**DE ES FR GB IT**

(71) Applicant: **DOW MITSUBISHI KASEI LIMITED**
**11-Kai, Seavans N-Kan, 2-1, Shibaura 1-chome**
**Minato-ku, Tokyo 105 (JP)**

(72) Inventor: **Tamaki, Yumiko**
**13-17, Moegino, Midori-ku**
**Yokohama-shi, Kanagawa 227 (JP)**
Inventor: **Katano, Hiroaki**
**1351-1, Miho-cho, Midori-ku**
**Yokohama-shi, Kanagawa 226 (JP)**
Inventor: **Yamaguchi, Shigeru**
**1723-7, Tsurumaki**
**Hatano-shi, Kanagawa 257 (JP)**

(74) Representative: **Raynor, John**
**W.H. Beck, Greener & Co 7 Stone Buildings**
**Lincoln's Inn**
**London WC2A 3SZ (GB)**

(54) Polyisocyanate composition.

(57) Storage stable high reactive polyisocyanate composition capable of withstanding a wide range of storage and handling conditions contain from 30 to 500 ppm of an organometallic based urethane catalyst and from 30 to 10,000 ppm of an acyl chloride compound based on the polyisocyanate compound. The composition can be utilized effectively in various spot applications such as spray foaming, painting for water-proofing, floor painting and paving, or the production of various polyurethane resins or foams.

EP 0 561 568 A1

This invention relates to a storage stable polyisocyanate composition. More particularly, the present invention relates to a storage stable and highly reactive polyisocyanate composition capable of being stored and handled under a wide range of conditions.

Polyurethanes prepared by reacting a polyisocyanate with an active hydrogen-containing compound such as a polyol in the presence of other additives are widely utilized in the production of various products for use in various fields. Polyurethanes are, for example, utilized in automobiles for seat cushions, steering wheels, instrument pads, headrests, bumpers and other interior or exterior parts, as electrical or heat insulators, for example in refrigerators, in display cases, as sealants for air conditioners, as architectural and civil engineering materials such as outer wall panels, heat-insulating storm doors, other doors, paints, materials for sealing, caulking, water-sealing, water-proofing and sound-dampening, structural heat insulators for pipes, tanks and ships, daily household articles such as beds, underlay for carpet, mattress, cushion, bedding and watch bands, or sports goods such as inner boots for skiing, shoe sole for training shoes and sneakers, artificial turf, wheels for roller skates and shopping carts. Polyurethanes are also utilized as fabrics as represented by "SPANDEX", fibers, adhesives or synthetic leathers.

Various attempts have been made to improve the storage stability of polyisocyanates, for example those described in Japanese Kokai Patent No. 61-69824, in which a specific organozirconium compound is employed as a reaction catalyst to prepare a NCO-terminated polyurethane prepolymer, and those described in EP-A-0516885 and EP-A-0517527, in which a specific amount of silicate compound or modified silicone oil is employed to improve storage stability at low temperatures. These approaches, however, are technologies only to improve storage stability of polyisocyanate compounds or compositions.

As described above, polyisocyanates and derivatives thereof are widely utilized in the production of many polyurethane products. For this reason, they are subject to a wide range of storage and handling conditions. There are few cases however in which the storage and handling conditions can be strictly controlled. In particular, the storage and handling conditions are not usually well-controlled in various spot constructions such as spray foaming, painting for water-proofing, floor painting of baseball grounds and stands, or paving. ,As a result, isocyanates often react with atmospheric moisture because of insufficient control of storage and handling conditions, producing urea compounds. The crystallization or film of the urea compound formed on the surface often has a bad influence on the injection of urethanes, on processing machines and on completed products.

Since the fron regulation came into being in 1990, the demand for highly reactive polyisocyanate compounds and derivatives thereof has been increasing. The use of such highly reactive polyisocyanate compounds enables the reduction of catalyst content in polyol premixtures comprising a polyol, a silicone surfactant, a blowing agent and other assistant agents. However, in spot constructions using highly reactive polyisocyanate compounds, urea compounds are often produced by the reaction of isocyanate with atmospheric moisture because of insufficient control of storage and handling conditions. It is therefore now particularly desirable to develop highly reactive polyisocyanate compounds and derivatives thereof, having a wide range of permissible storage and handling conditions.

We have discovered that this can be achieved by employing a polyisocyanate composition containing a specific amount of an organometallic based urethane catalyst and an acyl chloride compound. Accordingly, the invention provides a polyisocyanate composition which contains from 30 to 500 ppm by weight of an organometallic based urethane catalyst and from 30 to 10,000 ppm by weight of an acyl chloride compound, based on the polyisocyanate compound.

Suitable polyisocyanate compounds useful in the present invention include aliphatic polyisocyanates, alicyclic polyisocyanates, aromatic polyisocyanates, heterocyclic polyisocyanates and oligomers thereof, modified carbodiimides, modified polyols and modified allophanates. These compounds can be employed alone or as a mixture comprising at least two compounds. Suitable aliphatic polyisocyanates include, for example, ethylene diisocyanate, 1,4-tetramethylene diisocyanate, 1,6-hexamethylene diisocyanate, 1,2-dodecane diisocyanate, 2,2',4-trimethylhexamethylene diisocyanate, 2,4,4'-trimethyl- hexamethylene diisocyanate and dimeric acid diisocyanate. Suitable alicyclic polyisocyanates include, for example, cyclobutane-1,3-diisocyanate, cyclohexane-1,3- and -1,4- diisocyanate, isoferron diisocyanate, trans-cyclohexane-1,4- diisocyanate, 4,4'-methylene-bis(cyclohexylisocyanate), methylcyclohexane-2,4 diisocyanate, methylcyclohexane-2,6 diisocyanate and 1,3-(isocyanatemethyl)cyclohexane, Suitable aromatic polyisocyanates include, for example, m-phenylene diisocyanate, p-phenylene diisocyanate, 2,4-tolilene diisocyanate, 2,6-tolilene diisocyanate, m-xylilene diisocyanate, p-xylilene diisocyanate, 2,4'-diphenylmethane diisocyanate, 4,4'-diphenylmethane diisocyanate, m-tetramethylxylilene diisocyanate, p-tetramethyl- xylilene diisocyanate, thiophosphoric acid-tris(4-isocyanate phenylester), 3-isopropenyl-$\alpha,\alpha'$-dimethylbenzyl isocyanate and oligomer mixture thereof, or modified carbodiimides, modified polyols or modified allophanates. Preferable polyisocyanates include, for example, 2,4- and 4,4'-diphenylmethane diisocyanate and oligomer mixture thereof, modified carbodiimides or polyols diisocyanate, 1,6-hexamethylene diisocyanate, xylilene diisocyanate and oligomer mixture thereof, or modified carbodiimides or polyols thereof.

Suitable organometallic based urethane catalysts useful in the present invention include, for example, organic acid metal salts prepared from the reaction of an organic acid with a metal selected from tin, lead, copper, mercury, iron, cobalt, chromium, nickel, manganese and zinc. Suitable organotin based urethane catalysts include, for example, stannous acetate, stannous octoate, tin(II) 2-ethylcaproate, tin(II) oleate, dimethyltin dilaurate, dibutyltin-di(2-ethylhexoate), dabutyltin dioctoate, dibutyltin dilaurate, dibutyltin diacetate, dioctyltin dilaurate, dioctyltin bis-isothioglycolate, dioctyltin maleate polymer, tetrabutyl-1, 3-diacetoxystannoxane, dibutyltin dimaleate, dimethyltin dimaleate, dibutyltin dichloride, dimethyltin thiocarboxylate, dioctyltin thiocarboxylate, dimethyltin carboxylate, dibutyltin mercaptide, dioctyltin mercaptide and dimethyltin mercaptide. Suitable organolead based urethane catalysts include, for example, lead octanoate, lead naphthenate, lead nitrate and phenylmercury propionic acid salt. Other suitable organometallic based urethane catalysts include, for example, ferric chloride, ferric 2-ethylhexoate, cobalt 2-ethylhexoate, calcium octoate, calcium naphthenate, zinc octoate, zinc naphthenate, magnesium octoate and magnesium naphthenate. Preferable urethane catalysts in the stated compounds are, for example, organotin or organolead based urethane catalyst. Preferable organotin based catalysts include, for example, stannous acetate, stannous octoate, dibutyltin diacetate, dibutyltin dilaurate, dibutyltin thiocarboxylate, dibutyltin dimaleate, dioctyltin mercaptide, dioctyltin thiocarboxylate and 2-ethyltin caproate.

Suitable acylchloride compounds useful in the present invention include, for example, chlorinated compounds prepared from the reaction of chlorine with an acid such as monocarboxylic acid, dicarboxylic acid and aromatic carboxylic acid. Suitable monocarboxylic acids include, for example, acetic acid, propionic acid, butyric acid, valeric acid, methylethyl acetate, trimethyl acetate, caproic acid, heptonic acid, caprylic acid, capric acid, undecylic acid, lauric acid, myristic acid, palmitic acid, malgaric acid and stearic acid. Suitable dicarboxylic acids include, for example, oxalic acid, malonic acid, succinic acid, glutaric acid, adipic acid, pimelic acid, suberic acid, azelaic acid and sebacic acid. Suitable aromatic carboxylic acids include, for example, benzoic acid, toluic acid, p-penthyl benzoic acid, nitrobenzoic acid, phenyl acetate, cinnamic acid, dihydro cinnemic acid, phthalic acid, trimellitic acid and pyromerit acid. Preferable chlorinated compounds in the stated compounds include, for example, phthalic chloride, benzoic chloride, toluic chloride, adipic chloride and glutaric chloride.

The organometallic based urethane catalysts and acyl chloride compounds can be soluble or uniformly dispersed in the polyisocyanate compound, and the mixture gives a stable and normal composition. The catalyst content is from 30 to 500 ppm by weight, preferably from 50 to 200 ppm based on the polyisocyanate compound. The acyl chloride content is from 50 to 10,000 ppm by weight, preferably from 50 to 2,000 ppm, also based on the polyisocyanate compound. The addition of more than this amount of the catalyst causes only a slight increase of reactivity of the composition and is not economical. Although the storage stability of the composition is improved by the addition of the acyl chloride compound, the excess addition over the stated content is not desirable because it causes a decrease in the reactivity of the composition.

Suitable organic solvents to be employed as solvents for the polyisocyanate composition include, for example, carbonate esters such as ethylenecarbonate and propylenecarbonate, carboxylic esters such as phosphoric esters like chloroalkyl phosphate, dimethylmethyl phosphate, polymeric phosphates and brominated phosphate compounds, acetic ester, phthalic ester and trimellitic ester, aromatic hydrocarbons ("BTX") like benzene, toluene, xylene and chlorinated, nitrated or halogenated BTX, other esters, freons, halocarbons and chlorinated alkanes, other ethers such as ethylene glycol monoalkyl ethers and other aromatic or aliphatic solvents.

From the point of view of storage and handling, the polyisocyanate composition of the present invention has to be sufficiently controlled with respect to atmospheric moisture or other water because the reaction of the composition with moisture often produces an insoluble urea compound or causes an increase in viscosity of the composition.

The present invention is illustrated by the following Examples and Comparative Examples. It is to be understood, however, that the invention is not to be limited by the embodiments described in the following Examples. The term "AE" or "CE" in the following each Table means "Actual Example" or "Comparative Example" respectively.

Table 1 shows the characteristics of the various polyisocyanate compounds employed. Table 2 shows the organometallic based urethane catalysts and acyl chloride compounds employed.

REFERENCE EXAMPLES 1 TO 7 AND EXAMPLES 1 TO 7

The reactivity of the polyisocyanate compositions was evaluated using the following polyurethane formulation.

| POLYURETHANE COMPOSITION | | | |
|---|---|---|---|
| Polyisocyanate: | Polyisocyanate compound A (NCO content: 31.2%) | 157.1 | pbw |
| Polyol: | Propyleneoxide adducts of pentaerythritol (OH value: 410) | 100 | pbw |
| L-5420: | Silicon based surfactant (produced by Nihon Yunika) | 2 | pbw |
| Kaolizer No. 1: | Tetramethylhexamethylene diamine (produced by Kao) | 2.5 | pbw |
| Distilled water: | | 2.5 | pbw |
| Freon-11 | Trichloromonofluoromethane (produced by Asahi Glass) | 25 | pbw |

Table 3 shows the results of the evaluation when amounts of an organometallic based urethane catalyst, dibutyltin dilaurate ("DBTDL") of from 0 to 500 ppm were added to the polyisocyanate compound A.

Table 4 shows the results of the evaluation when an acyl chloride, isophthalic chloride of from 50 to 10,000 ppm was added to the polyisocyanate compound wherein the DBTDL content was 150 ppm. The reactivity of the polyurethane compositions was evaluated by sticking a wire, about 1 cm, into the composition during foaming and continually checking whether resin threads are made when the wire is pulled out from the foam. The figure described in the Table means the time from sticking the wire into the foam until pulling out the wire with resin threads.

EXAMPLES 8 TO 35 AND COMPARATIVE EXAMPLES 1 TO 5

The film forming property of the polyisocyanate composition employed was evaluated.by exposing the composition (about 30 ml) measured in a 100 ml beaker in the atmosphere under conditions of 23±1°C and 55±5% (relative humidity) and checking film formation by visual observation.

Table 5 shows film forming time for isophthalic chloride amounts of from 0 to 10,000 ppm with polyisocyanate compounds containing from of 60 to 500 ppm, DBTDL.

EXAMPLES 36 TO 38 AND COMPARATIVE EXAMPLES 6 TO 8

Table 6 shows further results relating to film forming, using from 0 to 1,000 ppm isophthalic chloride with polyisocyanate compounds B, C and D, containing 150 ppm, DBTDL.

EXAMPLES 39 TO 40 AND COMPARATIVE EXAMPLES 9 TO 10

Table 7 shows further results regarding film forming, using from 0 to 1,000 ppm isophthalic chloride with polyisocyanate compound A containing 150 ppm of various organometallic based urethane catalysts as shown in Table 2.

EXAMPLES 41 TO 46

Table 8 shows further film forming results using the various acyl chlorides shown in the Table 2 with polyisocyanate compound A containing 150 ppm DBTDL.

EXAMPLE 47

The chlorine content in the polyisocyanate composition containing an acyl chloride compound can be analyzed as free chlorine. The free chlorine content is determined by quantitative analysis after hydrolysis of the polyisocyanate. In other words, the free chlorine content is obtained by determining free hydrochloric acid with silver nitrate solution after adding a mixture of toluene, ethanol and water to specified amounts of polyisocyanate and heating the composition for 3 hours. Polyisocyanates prepared employing primary amines, phosgene and hydrochloric acid usually contain several hundred ppm chlorine. Various polyisocyanate compounds employed in the present invention also contain different amounts of chlorine. Based on this information, the relationship between chlorine content and film forming property was evaluated. The hydrolytic chlorine content of the polyisocyanate compound B employed in Example 47 was adjusted with an acyl chloride compound. Table 9 shows the results of the evaluation.

The present invention provides a storage stable and high reactive polyisocyanate composition having a wide range of storage and handling conditions. Accordingly, the polyisocyanate composition can effectively be utilized in various spot constructions such as spray foaming, painting for water-proofing, floor painting of baseball grounds and the like, and stands and paving, or the production of various polyurethane resins or foams.

Table 1

| | Modifying Method | Polyisocyanate | |
| --- | --- | --- | --- |
| | | NCO content (%) | Viscosity 25°C(cps) |
| A | MDI oligomer mixture | 31.2 | 113 |
| B | MDI oligomer mixture | 31.1 | 191 |
| C | MDI modified with carbodiimide | 29.1 | 31 |
| D | MDI oligomer mixture B : 50 %  MDI modified with polyol : 50 %  Polyol: polyetherpolyol having low  molecular weigth (NCO:23% ; 690cps) | 27.1 | 320 |

Table 2

| Compound | Product Name | Maker |
| --- | --- | --- |
| Dibutyltin Dilaurate (DBTDL) | U-100 | Nittou Kasei |
| Dibutyltin Dimercaptide (DBTDM) | UL-1 | Mitsubishi kasei Dow |
| Stannous Octoate (SnOct) | U-28 | Nittou Kasei |
| Isophthalic Chloride | - | Tokyo Kasei |
| Orthophthalic Chloride | - | Tokyo Kasei |
| Benzoic Chloride | - | Junsei Chemical |
| Glutaric Chloride | - | Tokyo Kasei |
| Adipic Chloride | - | Tokyo Kasei |
| p-Toluic Chloride | - | Kanto Chemical |

Table 3

| | Referential Example | | | | | | |
| --- | --- | --- | --- | --- | --- | --- | --- |
| | 1 | 2 | 3 | 4 | 5 | 6 | 7 |
| DBTDL (ppm) | 0 | 30 | 60 | 100 | 150 | 300 | 500 |
| Reactivity (sec) | 69 | 66 | 63 | 60 | 57 | 52 | 47 |

Table 4

| | Example | | | | | | |
|---|---|---|---|---|---|---|---|
| | 1 | 2 | 3 | 4 | 5 | 6 | 7 |
| Isophthalic Chloride (ppm) | 50 | 100 | 400 | 700 | 1000 | 5000 | 10000 |
| Reactivity (sec) | 50 | 57 | 56 | 56 | 57 | 65 | 89 |

Table 5

| | DBTDL (ppm) | Isophthalic Chloride (ppm) | Film Forming Time (hour) |
|---|---|---|---|
| CE  1 | 60 | 0 | 6 |
| AE  8 | 60 | 50 | 23 |
| AE  9 | 60 | 100 | 24 |
| AE 10 | 60 | 400 | 33 |
| AE 11 | 60 | 700 | 39 |
| AE 12 | 60 | 1000 | 45 |
| CE  2 | 100 | 0 | 3 |
| AE 13 | 100 | 50 | 23 |
| AE 14 | 100 | 100 | 24 |
| AE 15 | 100 | 400 | 27 |
| AE 16 | 100 | 700 | 33 |
| AE 17 | 100 | 1000 | 44 |
| CE  3 | 150 | 0 | 2 |
| AE 18 | 150 | 50 | 21 |
| AE 19 | 150 | 100 | 22 |
| AE 20 | 150 | 400 | 23 |
| AE 21 | 150 | 700 | 28 |
| AE 22 | 150 | 1000 | 35 |
| AE 23 | 150 | 5000 | 72 |
| AE 24 | 150 | 10000 | 120 |

Table 5 (Continued)

|  | DBTDL (ppm) | Isophthalic Chloride (ppm) | Film Forming Time (hour) |
|---|---|---|---|
| CE 4 | 300 | 0 | 0.5 |
| AE 25 | 300 | 100 | 21 |
| AE 26 | 300 | 400 | 22 |
| AE 27 | 300 | 700 | 27 |
| AE 28 | 300 | 1000 | 27 |
| AE 29 | 300 | 5000 | 66 |
| AE 30 | 300 | 10000 | 108 |
| CE 5 | 500 | 0 | 0.5 |
| AE 31 | 500 | 400 | 21 |
| AE 32 | 500 | 700 | 25 |
| AE 33 | 500 | 1000 | 25 |
| AE 34 | 500 | 5000 | 60 |
| AE 35 | 500 | 10000 | 96 |

Table 6

|  | Polyisocyanate Compound | Isophthalic Chloride (ppm) | Film Forming Time (hour) |
|---|---|---|---|
| CE 6 | B | 0 | 3 |
| AE 36 | B | 1000 | 96 |
| CE 7 | C | 0 | 0.2 |
| AE 37 | C | 1000 | 22 |
| CE 8 | D | 0 | 0.5 |
| AE 38 | D | 1000 | 11 |

Table 7

|  | Urethane Catalyst | Isophthalic Chloride (ppm) | Film Forming Time (hour) |
|---|---|---|---|
| CE 9 | DBTDM | 0 | 4 |
| AE 39 | DBTDM | 1000 | 31 |
| CE 10 | SnOct | 0 | 4 |
| AE 40 | SnOct | 1000 | 29 |

Table 8

|  | Acylchloride Compound | (ppm) | Film Forming Time (hour) |
|---|---|---|---|
| AE 41 | Isophthalic Chloride | 150 | 23 |
| AE 42 | Orthophthalic Chloride | 150 | 20 |
| AE 43 | Benzoic Chloride | 150 | 19 |
| AE 44 | Glutaric Chloride | 150 | 19 |
| AE 45 | Adipic Chloride | 150 | 21 |
| AE 46 | p-Toluic Chloride | 150 | 19 |

Table 9

|  | Isocyanate Compound | DBTDL (ppm) | Isophthalic Chloride (ppm) | Chlorine (ppm) | Film Forming Time (hour) |
|---|---|---|---|---|---|
| CE 3 | A | 150 | 0 | 270 | 2 |
| CE 6 | B | 150 | 0 | 160 | 3 |
| AE 47 | B | 150 | 310 | 270 | 23 |

## Claims

1. A polyisocyanate composition which contains from 30 to 500 ppm by weight of an organometallic based urethane catalyst and from 30 to 10,000 ppm by weight of an acyl chloride compound, based on the polyisocyanate compound.

2. The polyisocyanate composition of Claim 1 wherein the organometallic based urethane catalyst is an organotin or organolead based urethane catalyst.

3. The polyisocyanate composition of Claim 2 wherein the organotin based urethane catalyst is dibutyltin dilaurate, dibutyltin dimercaptide or stannous octoate.

4. The polyisocyanate composition of Claim 1 wherein the acyl chloride compound is a chlorinated compound prepared by the reaction of chlorine with a monocarboxylic acid, a dicarboxylic acid or an aromatic carboxylic acid.

5. The polyisocyanate composition of Claim 1 wherein the acyl chloride compound is isophthalic chloride, orthophthalic chloride, benzoic chloride, glutaric chloride, adipic chloride or p-toluic chloride.

**European Patent Office**

# EUROPEAN SEARCH REPORT

Application Number

EP 93 30 1893

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-1 328 600 (ALLIED CHEMICAL CORP.)<br>* page 1, right column, paragraph 1 -paragraph 2 *<br>* page 3, left column, paragraph 6 - right column, paragraph 2 *<br>* page 4, left column, paragraph 2 - right column, paragraph 1 *<br>--- | 1-5 | C07C263/18<br>C08G18/70 |
| A | FR-A-1 545 096 (MOBAY)<br>* page 1, right column, paragraph 2 *<br>* page 2, right column, paragraph 2 -paragraph 3 *<br>--- | 1 | |
| A | US-A-2 999 106 (R.M. PROSSER ET AL.)<br>* claims 1,5 *<br>* column 2, line 38 - line 55 *<br>--- | 1 | |
| A | US-A-2 437 867 (J.J. VERBANC)<br>* claims 1-8 *<br>* column 2, line 35 - column 3, line 22 *<br>----- | 1 | |

TECHNICAL FIELDS SEARCHED (Int. Cl.5)

C07C
C08G

The present search report has been drawn up for all claims

| Place of search | Date of completion of the search | Examiner |
|---|---|---|
| THE HAGUE | 10 JUNE 1993 | VAN PUYMBROECK M. A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0403)